# EUROPEAN PATENT APPLICATION

(11) **EP 2 641 561 A1**
(43) Date of publication of application: **25.09.2013**
(21) Application number: 13160094.2
(22) Date of filing: 20.03.2013
(51) Int. Cl.: A61B 19/00, A61B 1/00, A61B 5/06

(54) **System and method for determining camera angles by using virtual planes derived from actual images**

(30) Priority: 21.03.2012 US 201261613623 P; 28.02.2013 US 201313779793
(71) Applicant: Covidien LP, Mansfield, MA 02048 (US)
(72) Inventor: Pandey, Ashwini K., Wallingford, CT Connecticut 06492 (US)
(74) Representative: Soames, Candida Jane

(57) **Abstract**

An image output system (100) includes a plurality of surgical instruments (120, 122, 124; 210, 220, 230), where a positional and orientational relationship of each of the plurality of surgical instruments with respect to a target object (140) of a patient's body (150) is determined. The image output system further includes at least one video image capture unit (132, 134; 212, 222, 232) positioned on each of the plurality of surgical instruments and configured to selectively capture actual images (310; 410, 420, 430). A virtual image generator (270) is presented for selectively defining, generating, and assigning virtual images (320; 412, 422, 432) associated with the actual images relative to the target object (140) of the patient's body (150), the virtual images derived from each of the plurality of surgical instruments. An image processor is also presented for processing the actual images captured and the virtual images generated. Additionally, an image output device (102, 104; 290; 340) for displaying combinations (330; 440) of the actual images captured and the virtual images generated in a plurality of configurations is presented.

## Description

### CROSS REFERENCE TO RELATED APPLICATION

The present application claims the benefit of and priority to U.S. Provisional Application Serial No. 61/613,623, filed on March 21, 2012, the entire contents of which are incorporated herein by reference.

### BACKGROUND

### Technical Field

The present disclosure relates to endoscopic image output systems. More particularly, the present disclosure relates to systems and methods for combining actual images with virtual images derived therefrom for providing a surgeon with improved endoscopic orientation capabilities.

### Background of Related Art

Endoscopy refers to techniques used to inspect or to look into internal cavities or hollow structures. Endoscopic surgery, also called minimal access surgery, has become widely accepted because of clear-cut advantages such as a decreased postoperative morbidity, less pain, and a shorter hospitalization. Endoscopic surgery, however, is technically more demanding than 'classical open surgery' for several reasons such as smaller instruments, the limitation of the smaller entry ports, and limited visibility of the area operated upon. The learning curve of endoscopic surgery is much longer than expected a decade ago.

Moreover, endoscopy involves image guided surgical navigation, which is the process of planning minimally invasive surgical approaches and guiding surgical tools towards targets inside a patient's body with the help of anatomical imaging information obtained with techniques such as ultrasound, magnetic resonance, and various radiographic techniques. Such anatomical imaging information is useful because during a minimally invasive procedure, the surgical tools and the subcutaneous anatomy are not directly visible to the surgeon. With early image guided surgical techniques, the surgeon relied on his ability to accurately correlate two-dimensional slice-plane data with the three dimensionality of the patient in order to safely guide tools in the surgical field. The main drawbacks with this method were that it required abstract visualization by the surgeon in an attempt to develop an accurate mental picture of the interior anatomy, and that it did not provide feedback to the surgeon about the position of the surgical instruments during a procedure. Nevertheless, the combination of endoscopy and image guided surgery is interesting because it brings together the interior view of the endoscope and the exterior perspective of the image guided surgical system.

The value of using an image guidance system in conjunction with variable direction of view endoscopy is potentially much greater than for standard fixed-angle endoscopy. Firstly, such a combination would allow real and virtual image correlation over a much greater viewing range, which would mean improved approach planning, improved guidance capabilities, and improved procedures overall. Secondly, it would provide a significant betterment of viewing navigation with variable direction of view endoscopes. However, a problem introduced by variable direction of view endoscopes is that it is difficult for the surgeon to estimate the changing endoscopic line of sight, which has a variable relationship to the shaft axis, because the tip of the instrument is concealed during use. Acquiring an external estimate of where the endoscope is "looking" during a procedure is important as the surgeon tries to integrate preexisting knowledge of the anatomy with the viewing process.

Therefore, it should become apparent that there is a need for a method which provides at least the following capabilities: improved endoscopic orientation capabilities, global monitoring of endoscopic position and viewing direction, and improved surgical approach and procedure planning.

### SUMMARY

Accordingly, an image output system is provided. The image output system includes a plurality of surgical instruments, where a positional and orientational relationship of each of the plurality of surgical instruments with respect to a target object of a patient's body is determined. At least one video image capture unit is positioned on each of the plurality of surgical instruments and configured to selectively capture actual images. Additionally, an image generator for selectively defining, generating, and assigning virtual images associated with the actual images relative to the target object of the patient's body is provided, the virtual images derived from each of the plurality of surgical instruments. The image output system also includes an image processor for processing the actual images captured and the virtual images generated, and an image output device for displaying combinations of the actual images captured and the virtual images generated in a plurality of configurations.

In further embodiments, the actual images are superimposed on the virtual images. The actual images are images which correspond to an actual view of a region of interest and are captured in real time.

The plurality of instruments are endoscopes equipped for navigation through the patient's body. The video image capture unit may be a camera.

In yet another embodiment, the combinations of the actual images captured and the virtual images generated are used for registering and updating virtual image data continuously and in real time.

The virtual images are extracted from planar virtual surfaces and arranged in a manner corresponding to the actual images, such that the planar virtual surfaces are normal to a viewing direction of the at least one video image capture unit of the plurality of surgical instruments. Stated otherwise, virtual viewing points are arranged in a manner corresponding to actual viewing points provided by the at least one video image capture unit positioned on each of the plurality of surgical instruments.

The system is a fixed reference system relating actual views provided by the at least one video image capture unit positioned on each of the plurality of surgical instruments to the target object of the patient's body.

Additionally, an image output method is provided. The method includes selectively acquiring actual images from at least one video image capture unit positioned on each of a plurality of surgical instruments and determining a positional and orientational relationship of each of the plurality of surgical instruments with respect to a target object of the patient's body. The method further includes selectively acquiring virtual images from an image generator, the image generator selectively defining, generating, and assigning the virtual images associated with the actual images relative to a target object of the patient's body, the virtual images derived from each of the plurality of surgical instruments. The method also includes processing the actual images and the virtual images via an image processor and displaying combinations of the actual images captured and the virtual images generated via an image output device.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is an image capture unit viewing system including a plurality of cameras for receiving actual images and generating virtual images of an anatomical structure of a body therefrom, in accordance with the present disclosure;

FIG. 2 is a system diagram of an image output system, in accordance with the present disclosure;

FIG. 3A illustrates a method of combining and displaying actual images received and virtual images generated from a single image capture unit, in accordance with the present disclosure;

FIG. 3B is a method of combining and displaying actual images received and virtual images generated from multiple image capture units of a plurality of surgical instruments, in accordance with the present disclosure;

FIG. 4 illustrates a user interface for an image capture unit viewing system, in accordance with the present disclosure; and

FIG. 5 illustrates a plurality of surgical instruments, each having at least one image capture unit for receiving actual images from a body cavity and generating a plurality of virtual planes based on the actual images received to define positional and orientational relationships, in accordance with the present disclosure.

### DETAILED DESCRIPTION

The following detailed description illustrates the present disclosure by way of example, not by way of limitation of the principles of the present disclosure. This description will clearly enable one skilled in the art to make and use the present disclosure, and describes several embodiments, adaptations, variations, alternatives and uses of the present disclosure, including what is presently believed to be the best mode of carrying out the present disclosure.

Embodiments of the presently disclosed apparatus will now be described in detail with reference to the drawings, in which like reference numerals designate identical or corresponding elements in each of the several views. As used herein, the term "distal" refers to that portion of the tool, or component thereof which is further from the user while the term "proximal" refers to that portion of the tool or component thereof which is closer to the user.

The "actual images" may be images which are visually captured, in particular images which correspond to an actual view of a region of interest or which directly image reality, because they comprise images that are implemented specifically by video capture apparatuses or by an object lens and are captured in real time. The term "actual images" refers to all images such as may be seen by the human eye and/or by the human eye with the assistance of for example a camera or an object lens. The "actual images" come from the object and/or part of the patient's body being observed itself and not, like the virtual image data, described below, from a data set for the part of the patient's body.

The "virtual image data" may comprise image data which is captured before or during navigation by means of computed tomography, magnetic resonance tomography, an x-ray recording or fluoroscopic recording, a PET or SPECT recording or another medical imaging method. The "virtual image data" is data derived from a data set for the part of the patient's body being observed. The "virtual image data" may be referred to throughout the specification as virtual images, virtual planes, virtual objects, virtual spaces, virtual surfaces, virtual models, virtual views or virtual points.

In an exemplary embodiment of the present disclosure, the actual images are provided by a video image capture unit, in particular a camera or a camera light recorder (e.g., object lens or optical fiber end), such that the video image capture unit is arranged on or incorporated within an instrument.

In an exemplary embodiment of the present disclosure, a software program is run on a computer or other electronic device. The computer communicates electronically with an endoscope and a display device such as a monitor. The computer includes a graphics processing unit. The graphics processing unit is specifically designed to quickly perform the types of graphics related calculations required by the present disclosure. Other devices may be connected to the computer as appropriate for a given application.

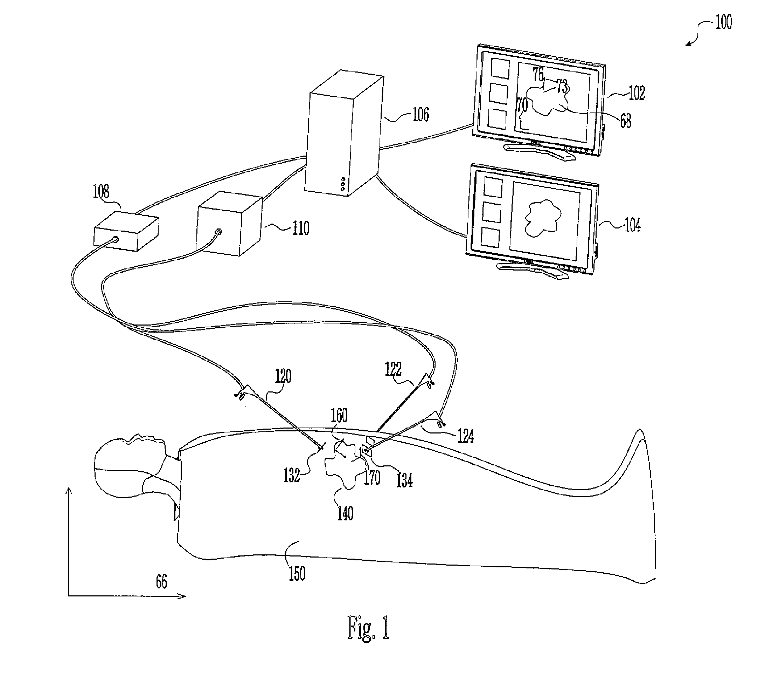

Referring initially to FIG. 1, an image capture unit viewing system including a plurality of cameras for receiving actual images and generating virtual images of an anatomical structure of a body therefrom, in accordance with the present disclosure is presented.

The image capture system 100 includes a plurality of video image capture units 132, 134 attached to each of the plurality of endoscopes 120, 122, 124. Endoscopes 120, 122 are connected to an image acquisition system 110, whereas endoscope 124 is connected to an actuator control unit 108, which are all in turn connected to a central control unit 106. Of course, any number of endoscopes may be connected to either the actuator control unit 108 or the image acquisition system 110. The central control unit 106 may be connected to a plurality of display units 102, 104.

Each of the plurality of endoscopes 120, 122, 124 may be enabled to create or develop or establish adjustable view vectors 160, 170, positioned with their distal ends in an anatomical structure 140 of a patient's body 150. Illumination for the anatomical structure 140 may be delivered through the plurality of endoscopes 120, 122, 124 from a standard light source (not shown). The plurality of endoscopes 120, 122, 124 may be equipped with actuators and sensors (not shown) that enable precise electromechanical control of the view vectors 160, 170. The user may control the view vectors 160, 170 through an input device such as a joystick or a keypad (not shown).

The central control unit 106 processes the user input and information about the current configuration of the plurality of endoscopes 120, 122, 124 to calculate the appropriate adjustment of the view vectors 160, 170 without changing the position of the plurality of endoscopes 120, 122, 124. The actuator control unit 108 controls the configuration of the plurality of endoscopes 120, 122, 124, while the image acquisition unit 110 receives image signals from the plurality of endoscopes 120, 122, 124 and adjusts them as needed before relaying them to the central control unit 106.

Endoscopic video images and additional relevant information are sent to display devices or units 102, 104. Light emitting diodes (or other transponders) on the plurality of endoscopes 120, 122, 124 are tracked by a set of cameras 132, 134. The central control unit 106 uses signals from the cameras 132, 134 to calculate the position of the plurality of endoscopes 120, 122, 124 in a global reference frame 66. A computer graphical model 68 of the interior anatomical structure 140, reconstructed from volumetric scan data obtained from an imaging procedure, has a model reference frame 70. By correlating the model reference frame 70 with the global reference frame 66, the central control unit 106 may calculate and display a graphical representation 73 obtained from the plurality of endoscopes 120, 122, 124 to illustrate their position relative to the anatomical structure 140 represented by a graphical model 68 on display device 104. The viewing direction is represented graphically as a view vector 76. The central control unit 106 keeps track of the orientation of the view vector 76 and uses the signals from the cameras 132, 134, which sense the emitters on the plurality of endoscopes 120, 122, 124 to calculate and display the relative positions of the plurality of endoscopes 120, 122, 124, the view vector 76, and the model 68.

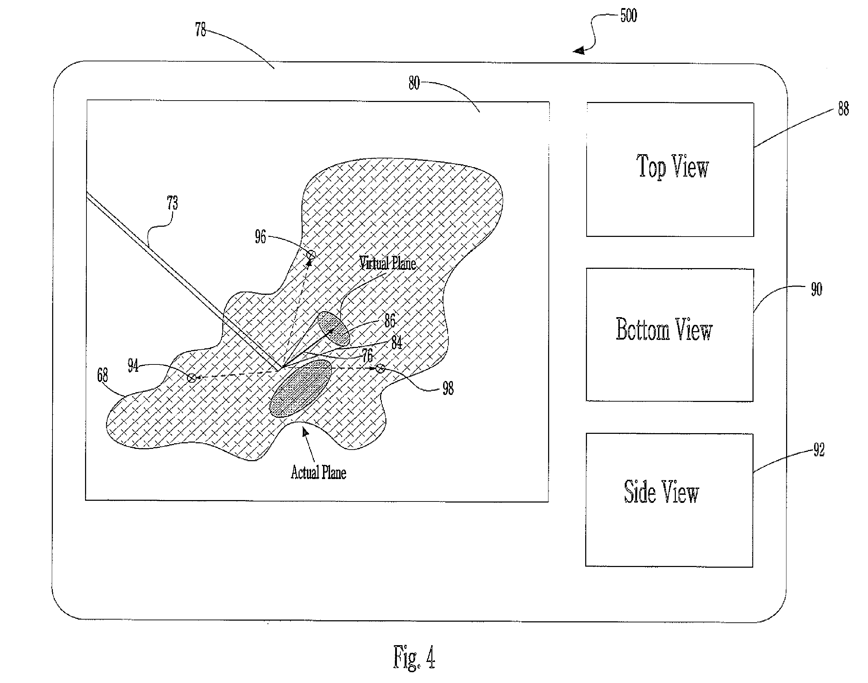

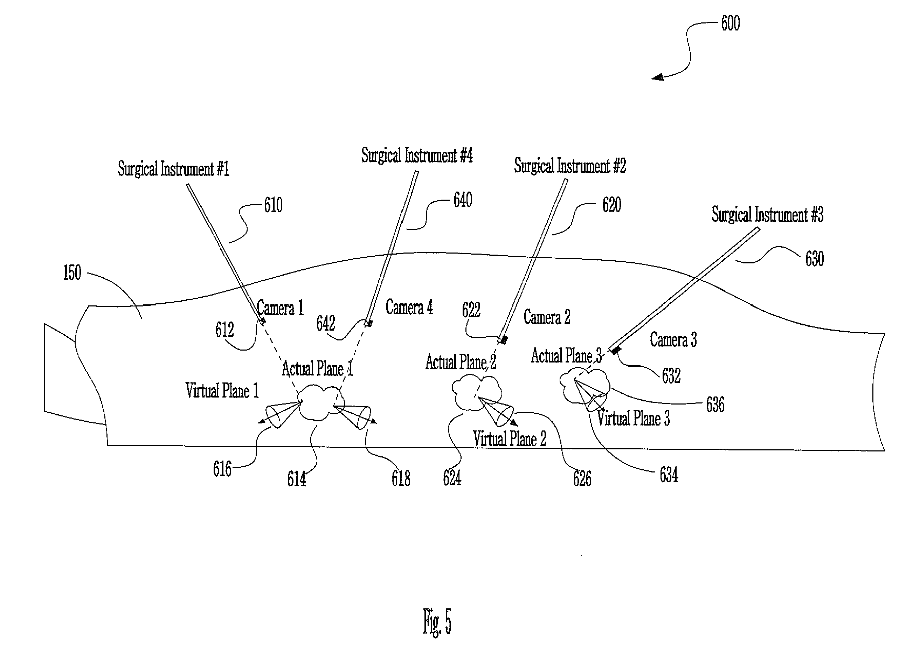

The relative positions of the plurality of endoscopes 120, 122, 124, their viewing directions, the anatomy, and the additional relevant information are presented to the user or surgeon via the display units 102, 104. The screen of the display units 102, 104 are organized into multiple sections, which display information about the endoscopic diagnosis or surgical procedure. A section of the display units 102, 104 is used to display the anatomical model 68 and graphical representations of the view vector 76, giving a global perspective of the endoscopic viewing direction and the location of the features seen in the endoscopic image relative to the surrounding anatomy. To aid the surgeon's spatial understanding, a representation of the endoscopic view cone (see FIGS. 4 and 5) is also displayed, and the orientation of the endoscopic image may be shown, indicating the up-direction of the actual image.

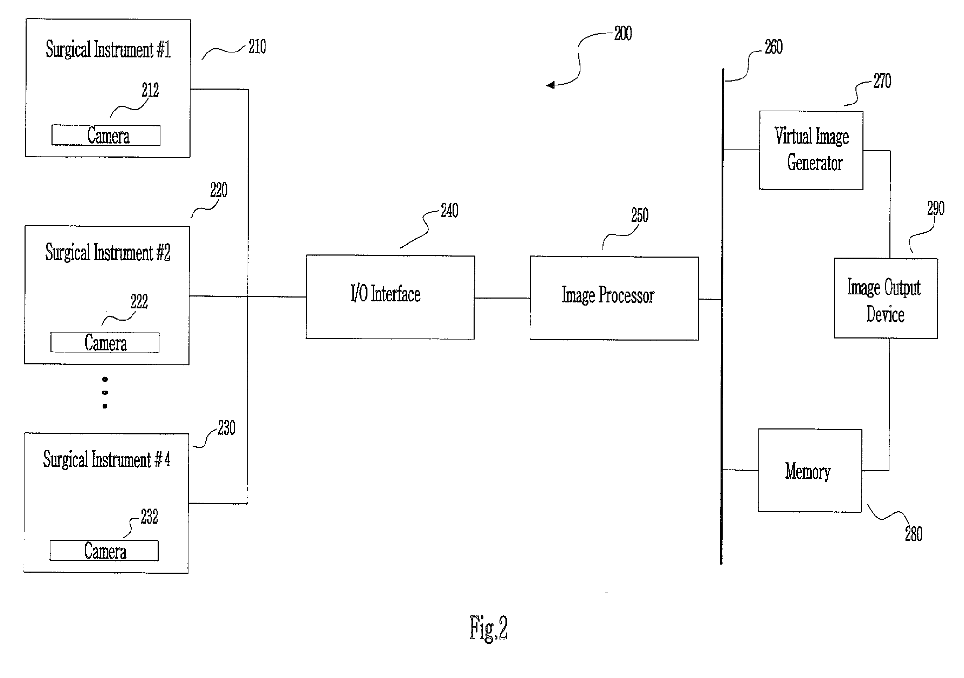

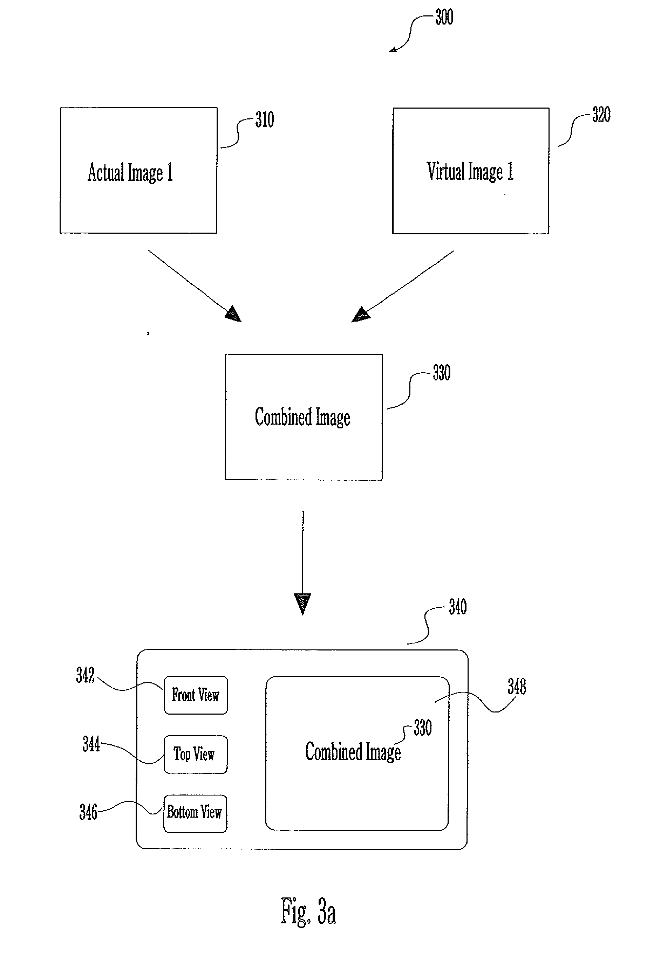

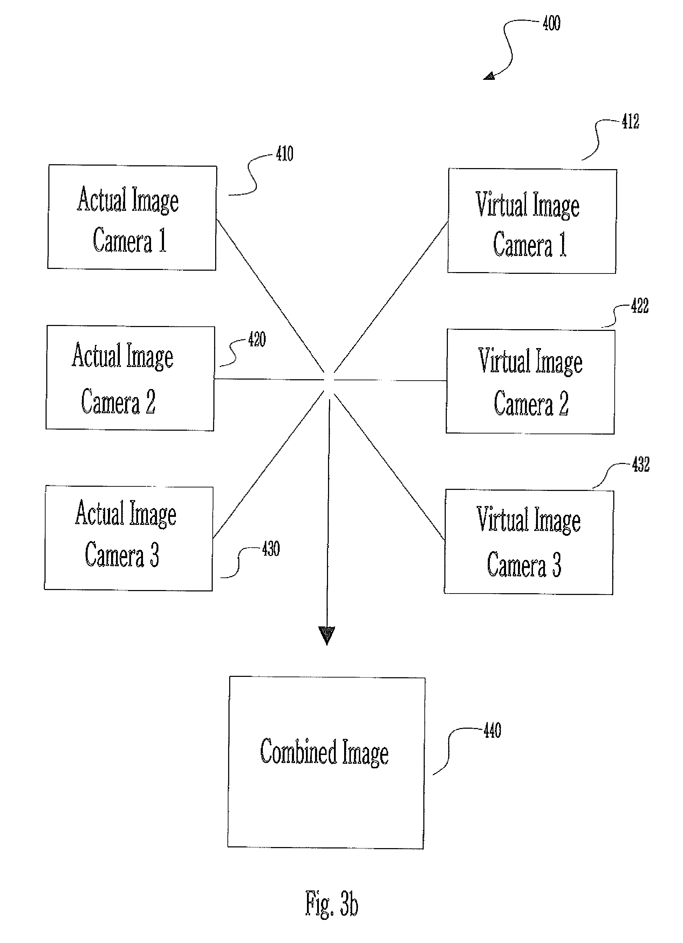

Therefore, the image output system 100 may include a plurality of endoscopes 120, 122, 124, where a positional and orientational relationship of each of the plurality of endoscopes 120, 122, 124 with respect to a target object 140 of a patient's body 150 is determined. At least one video image capture unit 132, 134 is positioned on each of the plurality of endoscopes 120, 122, 124 and is configured to selectively capture actual images of the target object 140 of the patient's body 150. The actual images are images, which correspond to an actual view of a region of interest and are captured in real time. The actual images obtained are to be combined with virtual images, as described below with reference to FIGS. 2, 3A, and 3B.

Additionally, the central control unit 106 may include a memory device for storing a program and other data. The video image capture units 132, 134 are so designated in broad terms as devices for providing appropriate images for processing in accordance with the present disclosure. For example, the video image capture units 132, 134 may be incorporated within an imaging device, such as a device incorporated in a CATSCAN, X-ray machine, an MRI or other device, or a stored image, or by communication with another computer or device by way of direct connection, a modulated infrared beam, radio, land line, facsimile, or satellite as, for example, by way of the World Wide Web or Internet, or any other appropriate source of such data. Data, such as actual images, received from the video image capture units 132, 134 may be stored in real time, continuously or in periodic intervals, in the memory device of the central control unit 106.

The memory device may be any type of storage unit. The term "storage unit" may refer to data storage. "Data storage" may refer to at least any article or material (e.g., a hard disk) from which information is capable of being reproduced, with or without the aid of any other article or device. "Data storage" may refer to at least the holding of data in an electromagnetic form for access by a computer processor. Primary storage is data in random access memory (RAM) and other "built-in" devices. Secondary storage is data on hard disk, tapes, and other external devices. "Data storage" may also refer to the permanent holding place for digital data, until purposely erased. "Storage" implies a repository that retains its content without power. "Storage" mostly means magnetic disks, magnetic tapes and optical discs (CD, DVD, etc.). "Storage" may also refer to non-volatile memory chips such as flash, Read-Only memory (ROM) and/or Electrically Erasable Programmable Read-Only Memory (EEPROM).

The display units 102, 104 may include a computer type display device using any suitable apparatus such as a cathode-ray kinescope tube, a plasma display, liquid crystal display, and so forth, or it may or may not include a device for rendering an image and may include a memory device or part of the memory device for storing an image for further processing, or for viewing, or evaluation, as may be convenient, or it may utilize a connection or coupling including such as are noted above in relation to the video image capture units 132, 134.

With reference to FIG. 2, a system diagram of an image output system, in accordance with the present disclosure is presented.

The system diagram 200 includes a plurality of surgical instruments 210, 220, 230. The first surgical instrument 210 includes a camera 212. The second surgical instrument 220 includes a camera 222. The nth surgical instrument 230 includes a camera 232. The surgical instruments 210, 220, 230 are operatively associated with an input/output (I/O) interface 240. The I/O interface 240 is operatively associated with an image processor 250. The image processor 250 is connected via a bus 260 to a virtual image generator 270, a memory 280, and an image output device 290.

The image processor 260 is configured to process the actual images captured and the virtual images generated by the virtual image generator 270. As used herein, the term "processor" may be used to refer to any type of computer, processor(s), or logic which may receive and process actual and virtual images detected by cameras positioned on or incorporated within a plurality of surgical instruments. Such a processor may include software for performing image processing of "actual images" and "virtual images" derived therefrom.

The virtual image generator 270 selectively defines, generates, and assigns virtual images associated with actual images received from the cameras 212, 222, 232 of the surgical instruments 210, 220, 230 relative to a target object 140 of a patient's body 150 (see FIG. 1).

The memory 280 is configured for storing a program and other data and has been described in detail above with reference to FIG. 1.

The image output device 290 may include any type of display means, as described above with reference to FIG. 1.

Therefore, in the present disclosure, multiple endoscopes or surgical instruments 210, 220, 230 are used. Each of the surgical instruments 210, 220, 230 includes at least one camera 212, 222, 232. Each of the surgical instruments 210, 220, 230 is capable of acquiring actual images of a target object in a patient's body. Based on the actual images obtained, one or more virtual planes or images are created by the virtual image generator 270 in association with the image processor 250. The actual images and the virtual images derived therefrom may be stored in a memory 280 and may be displayed on an image output device 290. A single image or multiple images may then be composed that combine the actual images and the virtual images derived therefrom (see FIGS. 3A, 3B).

With reference to FIG. 3A, a method of combining and displaying actual images received and virtual images generated therefrom from a single image capture unit, in accordance with the present disclosure is presented.

For example, the method 300 illustrates a first actual image 310 obtained from a camera mounted on or incorporated within a surgical instrument. Based on the first actual image 310, a virtual image generator produces a first virtual image 320. The first actual image 310 and the first virtual image 320 are combined to form a combined image 330. The combined image 330 is provided to, for example, an image output device 340. The image output device 340 displays the combined image 330 on a screen 348. Additionally, the image output device 340 may display several different views of the combined image 330. For instance, a front view 342, a top view 344, and a bottom view 346 may be generated and displayed in separate screens. It is noted that combining images may refer to superimposing actual images onto virtual images. Therefore, such method 300 may provide a surgeon with virtual planes from actual images obtained from the cameras in order to expand his/her viewing capabilities of the surgical site.

With reference to FIG. 3B, a method of combining and displaying actual images received and virtual images generated therefrom with the aid of multiple image capture units, in accordance with the present disclosure is presented.

The method 400 expands on the concept presented in the method 300 of FIG. 3A. In the method 400, the image output system includes three surgical instruments. The first surgical instrument has a first camera for capturing a first actual image 410. Based on the first actual image 410, an image generator generates a first virtual image 412. The second surgical instrument has a second camera for capturing a second actual image 420. Based on the second actual image 420, an image generator generates a second virtual image 422. The third surgical instrument has a third camera for capturing a third actual image 430. Based on the third actual image 430, an image generator generates a third virtual image 432.

The first actual image 410 is combined with the first virtual image 412, the second actual image 420 is combined with the second virtual image 422, and the third actual image 430 is combined with the third virtual image 432. All three combined images may then be combined into a single combined image 440. As such, a plurality of surgical instruments may be used to each capture at least one actual image, wherein the at least one actual image from each of the plurality of surgical instruments is used to create a respective virtual image via a virtual image generator. Then, all such images obtained from all the surgical instruments may be combined to form a single image 440. Therefore, a virtual representation that indicates which way each surgical instrument is oriented relative to the patient may be obtained. As such, a surgeon may view multiple virtual planes derived from multiple actual images, each of the actual images obtained from a plurality of surgical instruments. This results in an expanded field of view for the surgeon because he/she is able to view multiple target objects having multiple virtual planes, in addition to the actual planes received from the cameras.

Moreover, the combinations of the actual images captured and the virtual images generated may be used for registering and/or updating virtual image data continuously and in real time. Additionally, the virtual images may be extracted from planar virtual surfaces and arranged in a manner corresponding to the actual images, such that the planar virtual surfaces are normal to a viewing direction of the at least one video image capture unit of the plurality of surgical instruments.

With reference to FIG. 4, a user interface for an image capture unit viewing system, in accordance with the present disclosure is presented.

The user interface 500 of the display device 78 is organized into multiple sections, which display information about the endoscopic diagnosis or surgical procedure. A section of the screen 80 is used to display the anatomical model 68 and graphical representations of the endoscope 73 and the view vector 76, respectively, giving a global perspective of the endoscopic viewing direction and the location of the features seen in the endoscopic image relative to the surrounding anatomy. To aid the user's spatial understanding, a representation of the endoscopic view cone 84 is also displayed, and the orientation of the endoscopic image is shown by a marker 86, indicating the up-direction of the image. Three other sections or views 88, 90, 92 may show the orientation of the view vector 76 relative to the sagital, coronal, and axial slice planes containing the endoscope tip point. These slice planes change as the tip location of the endoscope is moved. Memory positions 94, 96, 98 indicate saved viewing locations to which the user may return. These memory positions 94, 96, 98 are fixed in the global coordinate system, so the endoscope may always find them, regardless of whether the body of the endoscope has moved since these positions were saved. Once again, this results in an expanded field of view for the surgeon because he/she is able to view a target object by deriving multiple virtual planes from one or more actual images seen by the cameras mounted on or incorporated within one or more surgical instruments.

With reference to FIG. 5, a plurality of surgical instruments, each having at least one image capture unit for receiving actual images from a body cavity and generating a plurality of virtual planes based on the actual images received to define positional and orientational relationships, in accordance with the present disclosure is presented.

The surgical system 600 depicts a first surgical instrument 610 having a first camera 612 for viewing a first target object 614. Based on the first actual plane, a first virtual plane 616 is created. Additionally, a fourth surgical instrument 640 having a fourth camera 642 for viewing the first target object 614 may be provided. Based on the first actual plane, a second virtual plane 618 is created. As such, a positional and orientational relationship is established between the first surgical instrument 610 and the fourth surgical instrument 640 with respect to the first target object 614 of the patient's body 150. Thus, the surgeon has multiple virtual planes 616, 618 in order to better view the first target object 614, the virtual planes 616, 618 derived from the actual plane.

Additionally, a second surgical instrument 620 may include a second camera 622 for viewing a second target object 624. Based on the second actual plane, a second virtual plane 626 is created. A third surgical instrument 630 may include a third camera 632 for viewing a third target object 634. Based on the third actual plane, a third virtual plane 636 is created. As such, a positional and orientational relationship is established between the second surgical instrument 620 with respect to the second target object 624 and the third surgical instrument 630 with respect to the third target object 634 of the patient's body 150.

Therefore, as shown in FIG. 5, the virtual images are extracted from planar virtual surfaces or planes, and are arranged in a manner corresponding to the actual images obtained from the cameras of the surgical instruments, such that the planar virtual surfaces or planes are normal to a viewing direction of the cameras of the surgical instruments. Stated otherwise, virtual viewing points are arranged in a manner corresponding to actual viewing points by the plurality of cameras positioned on or incorporated within the plurality of surgical instruments.

Consequently, once the surgeon has selected a target object, there are several options for the next step. For example, the surgeon may select the endoscope tip location, or may select an entry corridor or entry line, or may select to input the endoscopic field of view. After the user has selected any two of these three options, the central control unit 106 may determine the third. Typically, the entry corridor may be selected first because the surgeon's primary concern is to determine the entry path which provides adequate access to the target object in the safest way. Once the entry corridor and the object target have been determined, the central control unit 106 may, with standard computer graphics and machine vision algorithms, compute and display the virtual planes or virtual images acceptable for viewing the target object for a given endoscope.

With fixed viewing endoscopes, the selected entry corridor may not be possible for a given target object. In such cases, the central control unit 106 could calculate and display the range of acceptable entry corridors for a given endoscope if the user has input its field of view and viewing angle. It is only with omni-directional scopes that all entry corridors are possible, giving the surgeon complete freedom of selection. The virtual planes or virtual images available for a given target object depend on the field of view of the endoscope, the mobility of its view vector, and the shape of the surgical cavity. For example, the virtual planes or virtual images may be limited even for an omni-directional endoscope because of protruding tissue obstructing the target.

The central control unit 106 may also display possible combinations of entry corridors and tip locations for a given target object and endoscope type, giving the surgeon the opportunity to evaluate the combination which yields optimal positioning of the endoscope. It is also possible for the central control unit 106 to suggest favorable entry corridors for a given target object based on the endoscope type and anatomical data, making it possible for the user to insert the endoscope along the recommended path and then "look" in the direction of the target object upon arrival in the cavity. This type of obstacle avoidance path planning would include a minimal distance feature which calculates and displays a minimal entry distance. The image output device would graphically display the viewable area associated with each entry tip location on the model 68, giving the user instant feedback as to what the surgeon may expect to be able to see from various virtual view points. This includes indicating spots which would be occluded by intervening/overhanging tissue, and spots which would lie in blind zones of the endoscope based on the endoscope's insertion angle and tip position. From such actual images viewed, the virtual image generator 270 (see FIG. 2) may compute, produce, and/or generate one or more virtual images or virtual planes that are best suited for each surgical procedure. Such generated virtual planes or virtual images may be used, in association with the actual images to generate virtual fields of view of the surgeon in order to create improved endoscopic orientation capabilities.

In accordance with the present disclosure, the region of interest may be localized with navigation guidance, wherein the virtual images continuously augment or enhance the actual image data along the incision path. As a result, the system of the present disclosure is a fixed reference system relating actual views provided by the at least one video image capture unit positioned on each of the plurality of surgical instruments to the target object of the patient's body.

Moreover, stated otherwise, the exemplary embodiments of the present disclosure disclose a fixed reference system that relates a plurality of camera views obtained from a plurality of cameras mounted or incorporated within surgical instruments to a patient's anatomy, which would make it easier for a surgeon in understanding different perspectives offered by the plurality of cameras. The exemplary embodiments of the present disclosure are achieved by assigning a virtual plane associated with each camera by using, for example, gyroscopes, accelerometers or any such suitable technology so that the virtual plane is normal to the camera's direction. Such virtual planes from different cameras may be shown relative to each other, as well as the patient's anatomy on at least one output device. This provides the surgeon with a visual clue as to which plane provides him/her with the most desired view inside the patient's anatomy. By selecting one of the desired virtual planes by, for example, a mouse-click, the surgeon activates the camera to provide him with the best desired view.

In accordance with one exemplary embodiment of the present disclosure, the virtual image data may be weighted more heavily than the actual images when assembling the output image, such that the navigation-assisting information provided from the images, which is based on virtual image data, constitutes more than 50%, for example, more than 80% and up to 99.9%. The weighting will be dependent on the respective application.

It is possible to use the combination of the virtual image data and the actual images in order to positionally register the virtual image data, in particular for elastic image data registration (morphing). This combination may also be used for updating the virtual image data. The image material for assembling the image to be output, i.e., the image information, may be tested for relevance (and weighted) in the navigation system or by a specialized separate computer unit, such as the central control unit 106 (see FIG. 1), wherein less important image constituents are omitted from the image and/or more important image constituents are intensified or highlighted on the display units 102, 104 (see FIG. 1).

Computer program elements of the present disclosure may be embodied in hardware and/or software (including firmware, resident software, micro-code, etc.). The computer program elements of the present disclosure may take the form of a computer program product which may be embodied by a computer-usable or computer-readable storage medium comprising computer-usable or computer-readable program instructions, "code" or a "computer program" embodied in said medium for use by or in connection with the instruction executing system.

Within the context of this application, a computer-usable or computer-readable medium may be any medium which may contain, store, communicate, propagate or transport the program for use by or in connection with the instruction executing system, apparatus or device. The computer-usable or computer-readable medium may for example be, but is not limited to, an electronic, magnetic, optical, electromagnetic, infrared or semiconductor system, apparatus, device or medium of propagation, such as for example the Internet. The computer-usable or computer-readable medium could even for example be paper or another suitable medium on which the program is printed, since the program could be electronically captured, for example by optically scanning the paper or other suitable medium, and then compiled, interpreted or otherwise processed in a suitable manner. The computer program product and any software and/or hardware described here form the various means for performing the functions of the present disclosure in the example embodiment(s).

Moreover, the drawings and descriptions herein are necessarily simplified to depict the operation of the devices and illustrate various steps in the method. In use, the tissues may be manipulated by, and are frequently in contact with, the various tools and devices; however, for clarity of construction and operation, the figures may not show intimate contact between the tissues the tools and the devices.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of presently disclosed embodiments. Thus the scope of the embodiments should be determined by the appended claims and their legal equivalents, rather than by the examples given.

In particular with regard to the various functions performed by the elements (components, assemblies, devices, compositions, etc.) described above, the terms used to describe such elements (including any reference to a "means") are intended, unless expressly indicated otherwise, to correspond to any element which performs the specified function of the element described, i.e. which is functionally equivalent to it, even if it is not structurally equivalent to the disclosed structure which performs the function in the example embodiment(s) illustrated here.

Persons skilled in the art will understand that the devices and methods specifically described herein and illustrated in the accompanying drawings are non-limiting exemplary embodiments. The features illustrated or described in connection with one exemplary embodiment may be combined with the features of other embodiments. Such modifications and variations are intended to be included within the scope of the present disclosure. As well, one skilled in the art will appreciate further features and advantages of the present disclosure based on the above-described embodiments. Accordingly, the present disclosure is not to be limited by what has been particularly shown and described, except as indicated by the appended claims.

The invention may be described by reference to the following numbered paragraphs:-
1. An image output system comprising:
   a plurality of surgical instruments, where a positional and orientational relationship of each of the plurality of surgical instruments with respect to a target object of a patient's body is determined;
   at least one video image capture unit positioned on each of the plurality of surgical instruments and configured to selectively capture actual images;
   an image generator for selectively defining, generating, and assigning virtual images associated with the actual images relative to the target object of the patient's body, the virtual images derived from each of the plurality of surgical instruments;
   an image processor for processing the actual images captured and the virtual images generated; and
   an image output device for displaying combinations of the actual images captured and the virtual images generated in a plurality of configurations.
2. The image output system according to Paragraph 1, wherein the actual images are superimposed on the virtual images.
3. The image output system according to Paragraph 1, wherein the actual images are images which correspond to an actual view of a region of interest and are captured in real time.
4. The image output system according to Paragraph 1, wherein the plurality of instruments are endoscopes equipped for navigation through the patient's body.
5. The image output system according to Paragraph 1, wherein the video image capture unit is a camera.
6. The image output system according to Paragraph 1, wherein the combinations of the actual images captured and the virtual images generated are used for registering and updating virtual image data continuously and in real time.
7. The image output system according to Paragraph 1, wherein the virtual images are extracted from planar virtual surfaces and arranged in a manner corresponding to the actual images, such that the planar virtual surfaces are normal to a viewing direction of the at least one video image capture unit of the plurality of surgical instruments.
8. The image output system according to Paragraph 1, wherein virtual viewing points are arranged in a manner corresponding to actual viewing points provided by the at least one video image capture unit positioned on each of the plurality of surgical instruments.
9. The image output system according to Paragraph 1, wherein the system is a fixed reference system relating actual views provided by the at least one video image capture unit positioned on each of the plurality of surgical instruments to the target object of the patient's body.
10. A method for obtaining image data corresponding to interior portions of a patient's body, the method comprising:
   selectively acquiring actual images from at least one video image capture unit positioned on each of a plurality of surgical instruments;
   determining a positional and orientational relationship of each of the plurality of surgical instruments with respect to a target object of the patient's body;
   selectively acquiring virtual images from an image generator, the image generator selectively defining, generating, and assigning the virtual images associated with the actual images relative to a target object of the patient's body, the virtual images derived from each of the plurality of surgical instruments;
   processing the actual images and the virtual images via an image processor; and
   displaying combinations of the actual images captured and the virtual images generated via an image output device.
11. The method according to Paragraph 10, further comprising superimposing the actual images on the virtual images.
12. The method according to Paragraph 10, further comprising corresponding the actual images to an actual view of a region of interest, the actual images captured in real time.
13. The method according to Paragraph 10, wherein the plurality of instruments are endoscopes equipped for navigation through the patient's body.
14. The method according to Paragraph 10, wherein the video image capture unit is a camera.
15. The method according to Paragraph 10, further comprising registering and updating virtual image data continuously and in real time.
16. The method according to Paragraph 10, further comprising:
   extracting the virtual images from planar virtual surfaces; and
   arranging the virtual images in a manner corresponding to the actual images, such that the planar virtual surfaces are normal to a viewing direction of the at least one video image capture unit of the plurality of surgical instruments.
17. The method according to Paragraph 10, further comprising arranging virtual viewing points in a manner corresponding to actual viewing points provided by the at least one video image capture unit.
18. The method according to Paragraph 10, further comprising displaying an actual image by selecting a virtual image.
19. The method according to Paragraph 16, further comprising displaying an actual image by selecting a virtual image.

## Claims

1. An image output system comprising:
a plurality of surgical instruments, where a positional and orientational relationship of each of the plurality of surgical instruments with respect to a target object of a patient's body is determined;
at least one video image capture unit positioned on each of the plurality of surgical instruments and configured to selectively capture actual images;
an image generator for selectively defining, generating, and assigning virtual images associated with the actual images relative to the target object of the patient's body, the virtual images derived from each of the plurality of surgical instruments;
an image processor for processing the actual images captured and the virtual images generated; and
an image output device for displaying combinations of the actual images captured and the virtual images generated in a plurality of configurations.

2. The image output system according to Claim 1, wherein the actual images are superimposed on the virtual images.

3. The image output system according to Claim 1 or Claim 2, wherein the actual images are images which correspond to an actual view of a region of interest and are captured in real time.

4. The image output system according to any preceding Claim, wherein the plurality of instruments are endoscopes equipped for navigation through the patient's body; and/or wherein the video image capture unit is a camera; and/or wherein the combinations of the actual images captured and the virtual images generated are used for registering and updating virtual image data continuously and in real time.

5. The image output system according to any preceding Claim, wherein the virtual images are extracted from planar virtual surfaces and arranged in a manner corresponding to the actual images, such that the planar virtual surfaces are normal to a viewing direction of the at least one video image capture unit of the plurality of surgical instruments; and/or wherein virtual viewing points are arranged in a manner corresponding to actual viewing points provided by the at least one video image capture unit positioned on each of the plurality of surgical instruments.

6. The image output system according to any preceding Claim, wherein the system is a fixed reference system relating actual views provided by the at least one video image capture unit positioned on each of the plurality of surgical instruments to the target object of the patient's body.

7. A method for obtaining image data corresponding to interior portions of a patient's body, the method comprising:
selectively acquiring actual images from at least one video image capture unit positioned on each of a plurality of surgical instruments;
determining a positional and orientational relationship of each of the plurality of surgical instruments with respect to a target object of the patient's body;
selectively acquiring virtual images from an image generator, the image generator selectively defining, generating, and assigning the virtual images associated with the actual images relative to a target object of the patient's body, the virtual images derived from each of the plurality of surgical instruments;
processing the actual images and the virtual images via an image processor; and
displaying combinations of the actual images captured and the virtual images generated via an image output device.

8. The method according to Claim 7, further comprising superimposing the actual images on the virtual images.

9. The method according to Claim 7 or Claim 8, further comprising corresponding the actual images to an actual view of a region of interest, the actual images captured in real time.

10. The method according to any of Claims 7 to 9, wherein the plurality of instruments are endoscopes equipped for navigation through the patient's body.

11. The method according to any of Claims 7 to 10, wherein the video image capture unit is a camera.

12. The method according to any of Claims 7 to 11, further comprising registering and updating virtual image data continuously and in real time.

13. The method according to any of Claims 7 to 12, further comprising:
extracting the virtual images from planar virtual surfaces; and
arranging the virtual images in a manner corresponding to the actual images, such that the planar virtual surfaces are normal to a viewing direction of the at least one video image capture unit of the plurality of surgical instruments.

14. The method according to any of Claims 7 to 13, further comprising arranging virtual viewing points in a manner corresponding to actual viewing points provided by the at least one video image capture unit.

15. The method according to any of Claims 7 to 14, further comprising displaying an actual image by selecting a virtual image.
